(19) Europäisches Patentamt
European Patent Office
Office européen des brevets

(11) Veröffentlichungsnummer : **0 181 502**
**B1**

(12) # EUROPÄISCHE PATENTSCHRIFT

(45) Veröffentlichungstag der Patentschrift : **20.01.88**

(51) Int. Cl.[4] : **C 07 C 53/12**, C 07 C 51/54, B 01 J 31/16

(21) Anmeldenummer : **85112887.6**

(22) Anmeldetag : **11.10.85**

(54) **Verfahren zur Herstellung von Monocarbonsäureanhydriden.**

(30) Priorität : **07.11.84 DE 3440644**

(43) Veröffentlichungstag der Anmeldung : **21.05.86 Patentblatt 86/21**

(45) Bekanntmachung des Hinweises auf die Patenterteilung : **20.01.88 Patentblatt 88/03**

(84) Benannte Vertragsstaaten : **AT BE CH DE FR GB IT LI NL**

(56) Entgegenhaltungen :
**EP-A- 0 070 180**
**DE-A- 2 450 965**
**DE-A- 2 844 371**
**DE-A- 3 049 007**
**Journal of Molecular Catalysis, 2 (1977), 223-226**

(73) Patentinhaber : **HOECHST AKTIENGESELLSCHAFT**
**Postfach 80 03 20**
**D-6230 Frankfurt am Main 80 (DE)**

(72) Erfinder : **Luft, Gerhard, Prof. Dr.**
**Ludwigstrasse 141a**
**D-6109 Mühltal (DE)**
Erfinder : **Ritter, Gebhard, Dr.**
**Johann-Peter-Hebelstrasse 11**
**D-7601 Schutterwald/Baden (DE)**

EP 0 181 502 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von Monocarbonsäureanhydriden der allgemeinen Formel $(RCO)_2O$ durch Umsetzung eines Carbonsäureesters oder Dialkylethers der allgemeinen Formeln RCOOR bzw. ROR, wobei R jeweils denselben Alkylrest mit 1-4 C-Atomen bedeutet, mit Kohlenmonoxid in der Gasphase in Gegenwart von Jod oder Brom oder deren Verbindungen als Reaktionspromotor sowie in Gegenwart eines Trägerkatalysators bei Temperaturen von 130 bis 400 °C und Drücken von 1-150 bar.

Ein derartiges, in der Gasphase mit einem Trägerkatalysator arbeitendes Verfahren ist bereits aus der DE-Offenlegungsschrift 24 50 965 und der JP-Offenlegungsschrift Nr. 47921/1975 bekannt, welche die bei den Flüssigphase-Verfahren auftretenden Nachteile, z. B. die schwierige Abtrennung und Rückführung von suspendiertem und teilweise gelöstem Katalysator und ggf. Promotor, vermeiden.

In beiden Schriften werden jedoch Gasphase-Verfahren nur mit solchen festen Trägerkatalysatoren beschrieben, welche durch Imprägnierung des Trägermaterials mit Edelmetallverbindungen enthaltenden Katalysatorlösungen hergestellt wurden. Es ist aber auf diese Weise nicht möglich, z. B. Organostickstoff- oder Organophosphorverbindungen mit dreibindigem Stickstoff bzw. Phosphor im Trägerkatalysator zu fixieren, was sich im allgemeinen auf die Katalysatoraktivität und die Selektivität der Reaktion ungünstig auswirkt.

Vorliegende Erfindung vermeidet auch diese Mängel durch Anwendung sog. mehrfunktioneller Haftvermittler («Spacer»), in welche Promotoren der V. Hauptgruppe, z. B. Organylamine oder -phosphine, bereits integriert sind. Mit ihrer Hilfe ist es möglich, Nickelverbindungen fest an die Trägeroberfläche zu binden.

Im einzelnen ist die Erfindung nun dadurch gekennzeichnet, daß im Trägerkatalysator eine Organosiliciumverbindung mit Alkoxy- oder Halogengruppen sowie mit Organostickstoff-, Organophosphor-, Organoarsen-, Organoschwefel-, Mercapto- oder Thioethergruppen als mehrfunktioneller Haftvermittler einerseits an das Trägermaterial und andererseits an die Nickelverbindung gebunden ist.

Darüberhinaus kann das Verfahren der Erfindung wahlweise und bevorzugt dadurch gekennzeichnet sein, daß

a) der Trägerkatalysator zusätzlich Metallverbindungen aus der 1. bis 3. Hauptgruppe oder der 4. bis 7. Nebengruppe des Periodensystems der Elemente als Promotoren enthält ;

b) im Trägerkatalysator die Organosiliciumverbindung als mehrfunktioneller Haftvermittler einerseits an das Trägermaterial und andererseits abwechselnd an die Nickelverbindung und an eine Metallverbindung aus der 5. bis 7. Nebengruppe des Periodensystems der Elemente gebunden ist ;

c) der mehrfunktionelle Haftvermittler eine Organosiliciumverbindung folgender allgemeiner Formeln ist :

I. $R_n^1X_{3-n}Si-(CR_2^3)_m-Y$ oder
II. $R_n^1X_{3-n}Si-(CR_2^3)_m-CHY_2$ oder
III. $[R_n^1X_{3-n}Si-(CR_2^3)_m]_2Z$,

wobei

$X = -Cl, -Br$ oder $-OR^2$ ;
$Y = -NR_2^4$, ein stickstoffhaltiger Arylrest, $-PR_2^4$, $-AsR_2^4$, $-SR^4$ oder $-SH$ ;
$Z = -NR^4-$, $-PR^4-$, $-AsR^4-$ oder $-S-$ ;
$R^1 = C_1$ bis $C_5$-Alkyl ;
$R^2 = C_1$ bis $C_3$-Alkyl ;
$R^3 = -H$, $C_1$ bis $C_5$-Alkyl oder $-C_6H_5$ ;
$R^4 = C_1$ bis $C_6$-Alkyl, $C_5$ bis $C_8$-Cycloalkyl oder $-C_6H_5$ oder $C_6H_5CH_2-$, die ggf. mit Halogen-, Methoxy-, Ethoxy- oder $C_1$ bis $C_3$-Alkyl substituiert sind ;
$n = 0$ oder 1 oder 2 ;
$m = 0$ bis 8, vorzugsweise 1 bis 3.

d) der Trägerkatalysator ein anorganisches oxidisches Trägermaterial oder einen Aktivkohleträger enthält, deren restliche aktive Hydroxygruppen durch Veresterung oder Veretherung desaktiviert wurden ;

e) der Trägerkatalysator zusammen 0,01 bis 50 Gew%, vorzugsweise 0,1 bis 20 Gew%, Nickelverbindung, Haftvermittler und ggf. andere Nichtedelmetallverbindungen enthält ;

f) der Trägerkatalysator in einer Korngröße von 1 bis 20 mm eingesetzt wird.

Als Katalysatorträger kommen bevorzugt anorganische Oxide wie z. B. $SiO_2$, $Al_2O_3$, MgO, $TiO_2$, $La_2O_3$, $ZrO_2$, Zeolith, Ton, NiO, $Cr_2O_3$, $WO_3$ oder entsprechende Mischoxide, aber auch Aktivkohle infrage, welche BET-Oberflächen von 1-1 000 $m^2/g$, vorzugsweise 30-400 $m^2/g$, haben und stets noch OH-Gruppen aufweisen. Diese OH-Gruppen reagieren mit der oder den funktionellen Gruppen X des Haftvermittlers unter Bildung von Sauerstoffbrücken zwischen Träger und Haftvermittler. Die Promotoren

der 5. oder 6. Hauptgruppe sind im Haftvermittler chemisch gebunden und bilden selbst eine seiner funktionellen Gruppen, an welche die Nickelverbindungen, ggf. abwechselnd mit Metallverbindungen aus der 5. bis 7. Nebengruppe, insbesondere von Vanadium, Chrom oder Rhenium, angelagert werden. Hierbei können diese Nickel- und ggf. anderen Nichtedelmetallverbindungen Brücken zwischen den einzelnen, am Träger fixierten Haftvermittler-Molekülen bilden.

Es ist ein Vorteil des Verfahrens der Erfindung, daß die zur Steigerung der Katalysatoraktivität und der Selektivität notwendigen Promotoren aus der Hauptgruppe V oder VI des Periodensystems der Elemente eine funktionelle Gruppe Y oder Z in mehrfunktionellen Haftvermittlern bilden und somit bis zur maximalen Konzentration, die durch die Anzahl der OH-Gruppen auf der Trägeroberfläche bestimmt ist, fixiert werden können. Deshalb entfällt eine Abtrennung und Rückführung dieser z. B. Organostickstoff- oder Organophosphorpromotoren. Das Verfahren der Erfindung zur Herstellung von Monocarbonsäureanhydriden weist gegenüber den eingangs beschriebenen bekannten Verfahren, welche bereits in der Gasphase mit einem Trägerkatalysator arbeiten, höhere Katalysatoraktivitäten und Selektivitäten auf. Außerdem enthalten die erfindungsgemäß eingesetzten Trägerkatalysatoren keine teuren Edelmetalle aus der Gruppe VIII des Periodensystems der Elemente.

Das Verfahren der Erfindung dient insbesondere zur Herstellung von Essigsäureanhydrid aus Methylacetat oder Dimethylether in Gegenwart von Methyljodid oder Methylbromid als Reaktionspromotor. Als Reaktionspromotor kann auch HI, HBr oder allgemein RI oder RBr eingesetzt werden, wobei R einen Alkylrest mit 1-4 C-Atomen bedeutet.

In den allgemeinen Formeln für die Organosiliciumverbindungen, welche als Haftvermittler (Spacer) infrage kommen, bedeutet X vorzugsweise $—OR^2$ und insbesondere Methoxy und Ethoxy. Sofern n nicht ohnehin null ist, bedeutet $R^1$ bevorzugt einen unverzweigten Alkylrest, insbesondere Methyl, Ethyl oder Propyl.

Die Trägermaterialien wurden bereits genannt ; als Mischoxide kommen z. B. $Cr_2O_3$ - $Al_2O_3$, $WO_3$ - $Al_2O_3$, MgO - $Al_2O_3$, $SiO_2$ - $Al_2O_3$ oder $ZrO_2$ - $Al_2O_3$ infrage. Der Trägerkatalysator enthält bevorzugt, 0,01 bis 5 Gew% Nickel.

Als Nickelverbindungen kommen bei der Herstellung des Trägerkatalysators z. B. folgende Verbindungen infrage :

$$Ni(CO)_4,\ [P(C_6H_5)_3]_2Ni(CO)_2,\ NiCl_2,\ Ni(C_8H_{12})_2.$$

Als Metallverbindungen aus der 1. bis 3. Hauptgruppe oder der 4. bis 7. Nebengruppe des Periodensystems, vorzugsweise des Li, Na, Mg, Ca, Al, Ti, Zr, V, Cr, W, Re, können z. B. Hydroxide, Carbonate, Carbonyle, Hydride, Halogenide und andere Salze eingesetzt werden. Diese Metallverbindungen können z. B. als Lösung durch Imprägnierung auf den Katalysatorträger aufgebracht werden.

Zur Herstellung des erfindungsgemäß eingesetzten Trägerkatalysators muß zuerst der mehrfunktionelle Haftvermittler (Organosiliciumverbindung) bereitgestellt werden. Dieser ist entweder im Handel erhältlich oder kann nach oder in Analogie zu Literaturangaben dargestellt werden. Im allgemeinen wird jetzt an diese Haftvermittler, und zwar an die Promotorgruppe Y oder Z, welche ein Element aus der 5. oder 6. Hauptgruppe enthält, in an sich bekannter Weise eine der genannten Nickelverbindungen und ggf. eine der genannten Metallverbindungen aus der 5. bis 7. Nebengruppe angelagert.. Anschließend erfolgt die reaktive Anlagerung des nickelhaltigen Zwischenprodukts an die Hydroxygruppen des Trägermaterials unter Austritt einer Gruppe X als Verbindung XH (z. B. HCl, Hbr oder $R^2OH$). Dies wird durch 24- bis 100-Stündiges Erhitzen am Rückfluß der in einem unpolaren Lösemittel (z. B. Benzol, Toluol, Xylol) suspendierten Komponenten bis zur Entfärbung erreicht.

Andererseits kann man auch zuerst den mehrfunktionellen Haftvermittler (Organosiliciumverbindung) reaktiv unter Austritt einer Gruppe X als Verbindung XH an die Hydroxygruppen des Trägermaterials und erst anschließend die Nickelverbindung und ggf. eine der genannten Metallverbindungen aus der 5. bis 7. Nebengruppe an die Promotorgruppe Y oder Z des Zwischenprodukts anlagern.

Alle Einzelheiten ergeben sich aus der Katalysatorbeschreibung.

Besonders bei diskontinuierlichem und in der Anfahrphase bei kontinuierlichem Betrieb ist es zwecks Erhöhung der Selektivität und Unterdrückung von Nebenreaktionen sinnvoll, die restlichen OH-Gruppen auf der Oberfläche des Katalysatorträgers, welche nicht mit den funktionellen Gruppen X des Haftvermittlers reagiert haben, zu desaktivieren. Dies kann z. B. durch Silylierung mit Trimethylchlorsilan, Methylierung mit Methyljodid oder Acetylierung mit Essigsäureanhydrid geschehen.

Das Mengenverhältnis von Carbonsäureester bzw. Dialkylether und Jod(verbindung) oder Brom (verbindung) in der Reaktionszone kann innerhalb weiter Grenzen schwanken. Im allgemeinen beträgt die Menge des Carbonsäureesters und/oder Dialkylethers 1 bis 500 Mol, vorzugsweise 1 bis 100 Mol, je 1 Mol Jod(verbindung) oder Brom(verbindung). Die Temperatur der Reaktionszone wird so gewählt, daß das Reaktionsgemisch bei jedem beliebigem Umsatz gasförmig vorliegt. Bevorzugt wird die Temperatur zwischen 170 und 250 °C gewählt. Der bevorzugte Druck liegt zwischen 10 und 40 bar.

Die Verweilzeit des Reaktionsgemisches am festen Trägerkatalysator beträgt 1 bis 1 000 s, bevorzugt 1 bis 180 s. Die Umsetzung kann in einem vorzugsweise senkrecht angeordneten und mit Trägerkatalysator gefüllten Strömungsrohr oder auch in einem Rühr- oder Schüttelautoklaven erfolgen, der den Trägerkatalysator enthält. Man führt die Carbonylierung im allgemeinen unter praktisch wasserfreien

Bedingungen durch, doch ist die Anwesenheit geringer Wassermengen, wie sie in den handelsüblichen Ausgangsstoffen vorkommen, zulässig, sollte aber 1· Mol%, berechnet auf die Ausgangsstoffe, nicht übersteigen. Auch wird die Carbonylierung durch geringe Mengen Methanol in den Ausgangsstoffen nicht beeinträchtigt. Ebensowenig stört Wasserstoff, der in kleinen Mengen im handelsüblichen Kohlenmonoxid vorhanden sein kann.

Das aus der Carbonylierungszone abströmende Reaktionsgemisch ist gasförmig und enthält Kohlenmonoxid, Methyljodid, Essigsäureanhydrid, nicht-umgesetztes Methylacetat oder Dimethylether und ggf. geringe Mengen Essigsäure. Das gasförmige Reaktionsgemisch wird abgekühlt, wobei Essigsäureanhydrid und ggf. Essigsäure auskondensieren. Die nicht kondensierten Gase wie CO, $CH_3I$, Methylacetat oder Dimethylether werden in die Reaktionszone zurückgeführt, wobei die umgesetzten Anteile von Ester oder Ether sowie CO fortlaufend ersetzt werden. Die einfache Abtrennung der Anhydride durch Kühlung des abströmenden Reaktionsgemisches und Rückführung der nicht kondensierbaren Gase stellt einen wesentlichen Vorteil des erfindungsgemäßen Verfahrens dar, da dies ohne komplizierte Trennoperationen erfolgen kann. Der Trägerkatalysator wird nicht verunreinigt und verbleibt in der Reaktionszone, was den gesamten Verfahrensablauf bedeutend vereinfacht.

Beispiel 1

2 ml (1,86 g) Essigsäuremethylester, 0,5 ml (1,14 g) Methyljodid und 3,15 g des Katalysators

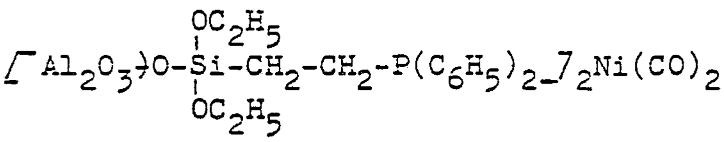

werden in einem 0,25 Liter fassenden Autoklaven aus korrosionsfreiem Edelstahl (Hastelloy C) mit Kohlenmonoxid bei 20 bar CO-Druck und 200 °C umgesetzt. Nach 1 h Reaktionsdauer ergibt sich eine Raum-Zeit-Ausbeute von 140 g $Ac_2O/g_{Ni} \cdot h$.

Die Ausbeute von $Ac_2O$, bezogen auf den eingesetzten Ester, beträgt 22 % bei einer Selektivität von 92 %.

Beispiel 2

1,86 g Dimethylether, 0,5 ml (1,14 g) Methyljodid und 3,15 g des Katalysators aus Beispiel 1 werden in dem Autoklaven aus Beispiel 1 mit Kohlenmonoxid bei 20 bar CO-Druck und 200 °C umgesetzt. Die Raum-Zeit-Ausbeute bezüglich Essigsäureanhydrid beträgt 30 g $Ac_2O/g_{Ni} \cdot h$. Der Versuch wurde über 5 h durchgeführt.

Die Ausbeute von $Ac_2O$, bezogen auf den eingesetzten Ether, beträgt 6 % bei einer Selektivität von 19 %.

Herstellung des Katalysators

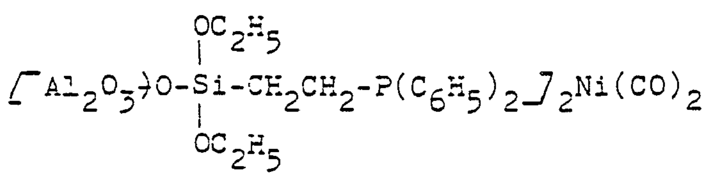

Aluminiumoxid wurde zwecks Aktivierung bei 200 °C und 0,1 bis 0,2 mbar 10 h getrocknet.

Die Herstellung des Katalysators wurde in Gegenwart von Stickstoff unter Ausschluß von Sauerstoff und Wasser durchgeführt, wobei sämtliche Reagenzien zuvor über Molekularsieb 4 A getrocknet worden waren.

Zu 3,2 g aktiviertem Aluminiumoxid (99 % $Al_2O_3$) mit einem Korndurchmesser von 3 mm, einer inneren Oberfläche nach BET von 125 $m^2/g$ und einem Porenvolumen von 0,9 ml/g gab man 65 mg (4,4 mg Ni) der Verbindung $[(C_2H_5O)_3Si—CH_2CH_2P(C_6H_5)_2]_2Ni(CO)_2$, gelöst in 40 ml Xylol, unter Rühren hinzu und brachte die Mischung zum Sieden. Nach 72 h Rückfluß war die gelbliche Lösung vollständig entfärbt. Nach Abtrennung des Lösemittels wurde der Katalysator in die Soxhlet-Apparatur überführt. Nach 12 h Soxhlet-Extraktion mit Benzol wurde der Katalysator bei 1,33 mbar und 85 °C acht Stunden getrocknet.

Zur Unterdrückung von Nebenreaktionen und Verbesserung der Selektivität wurde der Katalysator abschließend mit Trimethylchlorsilan behandelt.

$$Al_2O_3\}OH + Cl\text{-}Si(CH_3)_3 \xrightarrow[-HCl]{58\,^{\circ}C} Al_2O_3\}O\text{-}Si(CH_3)_3$$

Zu diesem Zweck wurde er bei Raumtemperatur mit Trimethylchlorsilan vollständig bedeckt. Diese Suspension wurde zum Sieden erhitzt und so lange unter Rückfluß gekocht bis keine Gasentwicklung mehr auftrat. Anschließend ließ man die Suspension abkühlen, trennte den Katalysator von der Flüssigkeit ab und trocknete ihn bei 85 °C und 1,33 mbar 12 Stunden lang.

Die eingeengten Lösemittel waren frei von Nickel. Der Nickelgehalt des so hergestellten Katalysators beträgt 0,13 Gew%.

Zur Herstellung der Zwischenverbindung $[(C_2H_5O)_3Si—CH_2CH_2P(C_6H_5)_2]_2Ni(CO)_2$ aus $(C_2H_5O)_3SiCH_2CH_2P(C_6H_5)_2$ und $Ni(CO)_4$ unter Abspaltung von CO-Gas siehe A.K. Smith et al., J. mol. Catal. 2 (1977) S. 223. Zur Herstellung von $(C_2H_5O)_3SiCH_2CH_2P(C_6H_5)_2$ aus Triethoxyvinylsilan und Diphenylphosphin unter UV-Belichtung siehe H. Niebergall, Makromol. Chem. 52 (1962), S. 218.

**Patentansprüche**

1. Verfahren zur Herstellung von Monocarbonsäureanhydriden der allgemeinen Formel $(RCO)_2O$ durch Umsetzung eines Carbonsäureesters oder Dialkylethers der allgemeinen Formeln RCOOR bzw. ROR, wobei R jeweils denselben Alkylrest mit 1-4 C-Atomen bedeutet, mit Kohlenmonoxid in der Gasphase in Gegenwart von Jod oder Brom oder deren Verbindungen als Reaktionspromotor sowie in Gegenwart eines Trägerkatalysators bei Temperaturen von 130 bis 400 °C und Drücken von 1-150 bar, dadurch gekennzeichnet, daß im Trägerkatalysator eine Organosiliciumverbindung mit Alkoxy- oder Halogengruppen sowie mit Organostickstoff-, Organophosphor-, Organoarsen-, Organoschwefel-, Mercapto- oder Thioethergruppen als mehrfunktioneller Haftvermittler einerseits an das Trägermaterial und andererseits an eine Nickelverbindung gebunden ist.

2. Verfahren nach Anspruch 1, dadurch gekennzeichnet, daß der Trägerkatalysator zusätzlich Metallverbindungen aus der 1. bis 3. Hauptgruppe oder der 4. bis 7. Nebengruppe des Periodensystems der Elemente als Promotoren enthält.

3. Verfahren nach Anspruch 1 oder 2, dadurch gekennzeichnet, daß im Trägerkatalysator die Organosiliciumverbindung als mehrfunktioneller Haftvermittler einerseits an das Trägermaterial und andererseits abwechselnd an die Nickelverbindung und an eine Metallverbindung aus der 5. bis 7. Nebengruppe des Periodensystems der Elemente gebunden ist.

4. Verfahren nach einem der Ansprüche 1-3, dadurch gekennzeichnet, daß der mehrfunktionelle Haftvermittler eine Organosiliciumverbindung folgender allgemeiner Formeln ist :

I. $R_n^1X_{3-n}Si—(CR_2^3)_m—Y$ oder
II. $R_n^1X_{3-n}Si—(CR_2^3)_m—CHY_2$ oder
III. $[R_n^1X_{3-n}Si—(CR_2^3)_m]_2Z$,

wobei

$X = —Cl$, $—Br$ oder $—OR^2$ ;
$Y = —NR_2^4$, ein stickstoffhaltiger Arylrest, $—PR_2^4$, $—AsR_2^4$, $—SR^4$ oder $—SH$ ;
$Z = —NR^4—$, $—PR^4—$, $—AsR^4—$ oder $—S—$ ;
$R^1 = C_1$ bis $C_5$-Alkyl ;
$R^2 = C_1$ bis $C_3$-Alkyl ;
$R^3 = —H$, $C_1$ bis $C_5$-Alkyl oder $—C_6H_5$ ;
$R^4 = C_1$ bis $C_6$-Alkyl, $C_5$ bis $C_8$-Cycloalkyl oder $—C_6H_5$ oder $C_6H_5CH_2—$, die ggf. mit Halogen-, Methoxy-, Ethoxy- oder $C_1$ bis $C_3$-Alkyl substituiert sind ;
$n = 0$ oder 1 oder 2 ;
$m = 0$ bis 8, vorzugsweise 1 bis 3.

5. Verfahren nach einem der Ansprüche 1-4, dadurch gekennzeichnet, daß der Trägerkatalysator ein anorganisches oxidisches Trägermaterial oder einen Aktivkohleträger enthält, deren restliche aktive Hydroxygruppen durch Veresterung oder Veretherung desaktiviert wurden.

6. Verfahren nach einem der Ansprüche 1-5, dadurch gekennzeichnet, daß der Trägerkatalysator zusammen 0,01 bis 50 Gew%, vorzugsweise 0,1 bis 20 Gew%, Nickelverbindung, Haftvermittler und ggf. andere Nichtedelmetallverbindungen enthält.

7. Verfahren nach einem der Ansprüche 1-6, dadurch gekennzeichnet, daß der Trägerkatalysator in einer Korngröße von 1 bis 20 mm eingesetzt wird.

**Claims**

1. Process for making monocarboxylic anhydrides of the general formula $(RCO)_2O$ by reacting a carboxylic acid ester of the general formula RCOOR or dialkylether of the general formula ROR, formulae in which R stands for one and the same alkyl group having from 1 to 4 carbon atoms, with carbon monoxide in gas phase in the presence of iodine or bromine or their compounds as a reaction promoter

and also in the presence of a carrier-supported catalyst, at temperatures of 130 to 400 °C and under pressures of 1 to 150 bars, characterized in that, in the carrier-supported catalyst, an organosilicon compound containing an alkoxy or halogen group and also an organonitrogen, organophosphorus, organoarsenic, organosulfur, mercapto or thioester group as a polyfunctional adhesion promoter is linked to the carrier material and also to a nickel compound.

2. Process as claimed in claim 1, wherein the carrier-supported catalyst additionally contains as a promoter a metal compound selected from the 1st through 3rd principal groups or the 4th through 7th subgroups of the Periodic System of the elements.

3. Process as claimed in claim 1 or 2, wherein the organosilicon compound as the polyfunctional adhesion promoter in the carrier-supported catalyst is linked to the carrier material and alternately also to the nickel compound and a metal compound selected from the 5th through 7th subgroups of the Periodic System of the elements.

4. Process as claimed in any of claims 1 to 3, wherein the polyfunctional adhesion promoter is an organosilicon compound corresponding to one of the following general formulae :

I. $R_n^1X_{3-n}Si-(CR_2^3)_m-Y$ or
II. $R_n^1X_{3-n}Si-(CR_2^3)_m-CHY_2$ or
III. $[R_n^1X_{3-n}Si-(CR_2^3)_m]_2Z$

in which

X stands for $-Cl$, $-Br$ or $-OR^2$ ;
Y stands for $-NR_2^4$, a nitrogen-containing aryl group, $-PR_2^4$, $-AsR_2^4$, $-SR^4$ or $-SH$ ;
Z stands for $-NR^4-$, $-PR^4-$, $-AsR^4-$ or $-S-$ ;
$R^1$ stands for a $C_1$ to $C_5$-alkyl ;
$R^2$ stands for a $C_1$ to $C_3$-alkyl ;
$R^3$ stands for $-H$, a $C_1$ to $C_5$-alkyl or $-C_6H_5$ ;
$R^4$ stands for a $C_1$ to $C_6$-alkyl, $C_5$ to $C_8$-cycloalkyl or $-C_6H_5$ or $C_6H_5CH_2$-optionally substituted with a halogen, methoxy, ethoxy or $C_1$ to $C_3$-alkyl.
n stands for 0 or 1 or 2 ;
m stands for 0 through 8, preferably 1 to 3.

5. Process as claimed in any of claims 1 to 4, wherein the carrier-supported catalyst contains an inorganic oxidic carrier or an active carbon carrier, the residual active hydroxy groups of which are inactivated by esterification or etherification.

6. Process as claimed in any of claims 1 to 5, wherein the carrier-supported catalyst contains altogether 0.01 to 50 wgt%, preferably 0.1-20 wgt% nickel compound, adhesion promoter and optionally further non noble metal compounds.

7. Process as claimed in any of claims 1 to 6, wherein the carrier-supported catalyst is used in form of particles with a size of 1 to 20 mm.

**Revendications**

1. Procédé de préparation d'anhydrides monocarboxyliques de formule générale $(RCO)_2O$ par réaction d'un ester carboxylique de formule générale RCOOR ou d'un éther dialkylique de formule générale ROR, dans lesquelles R représente chaque fois le même groupe alkyle en $C_1$-$C_4$, avec le monoxyde de carbone en phase gazeuse, en présence d'iode ou de brome ou de leurs composés comme promoteur de réaction, ainsi qu'en présence d'un catalyseur sur support à des températures de 130-400 °C et sous des pressions de 1-150 bars, caractérisé en ce que, dans le catalyseur sur support, un composé organosilicié contenant des groupes alcoxyles ou halogénés ainsi que des groupes organoazotés, organophosphorés, organoarséniés, organosoufrés, des groupes mercapto ou thioéthers comme auxiliaire de fixation polyfonctionnel est lié au support d'une part et à un composé de nickel d'autre part.

2. Procédé selon la revendication 1, caractérisé en ce que le catalyseur sur support contient comme promoteurs des composés métalliques supplémentaires appartenant aux groupes principaux 1-3 ou aux sous-groupes 4-7 de la Classification Périodique des Eléments.

3. Procédé selon la revendication 1 ou 2, caractérisé en ce que le composé organosilicié servant d'auxiliaire de fixation polyfonctionnel est lié dans le catalyseur sur support au support d'une part et, alternativement, au composé de nickel, et à un composé métallique appartenant aux sous-groupes 5-7 de la Classification Périodique des éléments d'autre part.

4. Procédé selon l'une des revendications 1-3, caractérisé en ce que l'auxiliaire de fixation polyfonctionnel est un composé organosilicié répondant à l'une des formules générales suivantes :

I. $R_n^1X_{3-n}Si(CR_2^3)_m-Y$ ou
II. $R_n^1X_{3-n}Si(CR_2^3)_m-CHY_2$ ou
III. $[R_n^1X_{3-n}Si-(CR_2^3)_m]_2Z$

dans lesquelles

X = $-Cl$, $-Br$ ou $-OR^2$ ;
Y = $-NR_2^4$, un groupe aryle azoté, $-PR_2^4$, $-AsR_2^4$, $-SR^4$ ou $-SH$ ;
Z = $-NR^4-$, $-PR^4-$, $-AsR^4-$ ou $-S-$ ;

$R^1$ = alkyle en $C_1$-$C_5$ ;
$R^2$ = alkyle en $C_1$-$C_3$ ;
$R^3$ = —H, alkyle en $C_1$-$C_5$ ou —$C_6H_5$ ;
$R^4$ = alkyle en $C_1$-$C_6$, cycloalkyle en $C_5$-$C_8$ ou —$C_6H_5$ ou $C_6H_5CH_2$—, éventuelement substitués par un halogène, un groupe méthoxy, éthoxy ou alkyle en $C_1$-$C_3$ ;
n = 0, 1 ou 2 ;
m = 0 à 8, de préférence 1 à 3.

5. Procédé selon l'une des revendications 1-4, caractérisé en ce que le catalyseur sur support contient un support d'oxyde inorganique ou un support de charbon actif dont les groupes hydroxyles actifs résiduaires ont été désactivés par estérification ou éthérification.

6. Procédé selon l'une des revendications 1-5, caractérisé en ce que le catalyseur sur support contient au total 0,01-50 % en poids, de préférence 0,1-20 % en poids, de composé de nickel, d'auxiliaire de fixation et, éventuellement, d'autres composés de métaux non nobles.

7. Procédé selon l'une des revendications 1-6, caractérisé en ce que l'on utilise le catalyseur sur support à une granulométrie de 1-20 mm.